# EUROPEAN PATENT SPECIFICATION

(11) **EP 3 065 567 B1**
(45) Date of publication and mention of the grant of the patent: **25.12.2019**
(21) Application number: 14799698.7
(22) Date of filing: 06.11.2014
(51) Int. Cl.: A23L 33/10, A61K 9/16, C07C 403/24

(54) **PROCESS FOR THE PURIFICATION OF ASTAXANTHIN**
VERFAHREN ZUR REINIGUNG VON ASTAXANTHIN
PROCESSUS DE PURIFICATION D'ASTAXANTHINE

(30) Priority: 07.11.2013 US 201361901136 P; 12.11.2013 CH 18902013
(43) Date of publication of application: 14.09.2016
(73) Proprietor: DSM IP Assets B.V., 6411 TE Heerlen (NL)
(72) Inventor: KARRER, Philippe, 4303 Kaiseraugst (CH); WUESTENBERG, Bettina, 4303 Kaiseraugst (CH)
(74) Representative: Steck, Melanie
(86) International application number: PCT/EP2014/073954
(87) International publication number: WO 2015/067705

(56) References cited:
- EP-A1- 0 633 258
- WO-A1-01/19383
- WO-A1-2007/128574
- WO-A1-2009/144329
- WO-A1-2010/058235
- WO-A2-2007/072529
- CN-A- 103 044 304
- AMBATI RANGA RAO ET AL: "Stabilization of astaxanthin in edible oils and its use as an antioxidant", JOURNAL OF THE SCIENCE OF FOOD AND AGRICULTURE, vol. 87, no. 6, 30 April 2007 (2007-04-30) , pages 957-965, XP55120279, ISSN: 0022-5142, DOI: 10.1002/jsfa.2766

## Description

The object of the invention is to provide a synthetic food-grade astaxanthin (AXN) which is not known so far. "Food-grade" within the present invention means "suitable for human consumption". Such a food-grade synthetic AXN thus fulfills all regulatory requirements concerning purity and can be used in the commercial production of food and dietary supplements.

AXN can be manufactured by chemical synthesis (see EP-A 908 449, WO 2005/087720, EP-A 733 619), it can be produced by fermentation (see EP-A 543 023) and isolated from natural sources such as shell waste (see JP-A 11-049 972) or algae *Haematococcus pluvialis* (see GB-A 2,301,587), where the isolated esters would have to be cleaved to AXN itself.

AXN isolated from natural sources is already used in products for human consumption: see e.g. US 2009/0297492, where AXN is described as improving the cognitive performance; US 2009/0018210, where AXN is described as promoting fat degradation; EP-A 1 867 327, where AXN is described for preventing a neurodegenerative disease - to name only a few. So far, however, synthetic AXN was not used in products for human consumption.

Synthetic AXN is of standardized quality compared to AXN from natural sources since it is much easier to use a standardized procedure for its chemical synthesis and purification than being dependent from the varying quality of natural AXN resulting by using natural sources which vary also in their composition. Thus, in the context of the present invention the term "AXN" means "synthetic AXN".

Chemical processes for the manufacture of AXN are often carried out in halogenated hydrocarbons such as dichloromethane (see e.g. WO 2011/095571) since AXN and its precursors have a high solubility in these solvents though there were intentions in the past to avoid the use of such solvents (see e.g. EP-A 908 449 and WO 2005/087720). There are also purification methods that use chloroform (see JP-A 07118226). The thus obtained AXN is then often crystallized from lower alkanols since it is hardly soluble in such solvents.

It was therefore an object of the present invention to provide a synthetic AXN which is of reliable quality, easy to obtain also in an industrial scale and suitable for human consumption.

Thus, it was an object of the present invention to provide a food-grade synthetic AXN.

There exist already processes for the purification of astaxanthin:
In example 11 of WO 2007/072529 the synthesis of astaxanthin is performed in isopropanol. The obtained product is recrystallized in three successive rounds: one with methylene chloride, one with methanol and one with heptane. Hereby all astaxanthin is dissolved.

According to WO 2007/128574 (see page 9, line 25) the filter cake of astaxanthin is washed with methanol.

According to EP-A 633 258 (see example 2) the astaxanthin crystals have been washed with heptane.

The object of the present invention, to provide a food-grade synthetic AXN, is met by the process of the present invention which yields an AXN with extremely low solvent levels, preferably with extremely low levels of dichloromethane, thus rendering this AXN suitable for human consumption.

Thus, the present invention is especially directed to provide a synthetic AXN with a content of dichloromethane ≤ 250 ppm, preferably with a content of dichloromethane ≤ 200 ppm, more preferably with a content of dichloromethane ≤ 100 ppm, even more preferably with a content of dichloromethane ≤ 50 ppm, ≤ 35 ppm, ≤ 30 ppm, ≤ 25 ppm, ≤ 20 ppm, most preferably with a content of dichloromethane ≤ 10 ppm.

In an especially preferred embodiment of the present invention the synthetic AXN has a content of dichloromethane in the range of between 0 and 100 ppm, preferably in the range of between 10 and 100 ppm.

Preferably the synthetic AXN obtained by the processes according to the present invention has a content of methanol ≤ 500 ppm, preferably it has a content of methanol ≤ 350 ppm, more preferably it has a content of methanol ≤ 250 ppm, even more preferably it has a content of methanol ≤ 150 ppm, ≤ 100 ppm, most preferably it has a content of methanol ≤ 50 ppm, ≤ 20 ppm, ≤ 10 ppm, ≤ 5 ppm.

In an especially preferred embodiment of the present invention the synthetic AXN has a content of methanol in the range of between 0 and 50 ppm, preferably in the range of between 0 and 20 ppm, more preferably in the range of 0 to 10 ppm, most preferably in the range of 0 to 5 ppm.

In a further preferred embodiment, the synthetic AXN has a content of dichloromethane < 50 ppm and a content of methanol < 100 ppm.

The synthetic AXN may especially be used in dietary supplements. For these purposes it is often provided in the form of an oily suspension or a powdery formulation such as a beadlet, thus protecting the synthetic AXN from degradation.

Thus, by the present invention it is possible to provide a human dietary supplement comprising astaxanthin manufactured by chemical synthesis (i.e. synthetic astaxanthin), free from astaxanthin mono- and diesters, and comprising ≤ 250 ppm of dichloromethane. Preferably these human dietary supplements comprising this synthetic AXN, free from AXN mono- and diesters, comprise ≤ 200 ppm of dichloromethane, ≤ 100 ppm of dichloromethane, ≤ 50 ppm of dichloromethane. More preferably these human dietary supplements comprising synthetic food-grade AXN, free from AXN mono- and diesters, comprise dichloromethane in the range between 0 and 100 ppm, preferably in the range between 10 and 100 ppm.

Moreover, the present invention can provide human dietary supplements comprising synthetic food-grade AXN, free from AXN mono-and diesters and with the reduced levels of dichloromethane and methanol as give above including all preferred ranges and combinations given.

Since it is possible with the processes of the present invention to significantly reduce the content of halogenated organic solvents such as dichloromethane, there is no need any more to use a chemical synthesis, where the use of such solvents is avoided.

### Processes

Such a synthetic food-grade AXN may be obtained by the following process which is an object of the present invention.

Process 1: A process for the manufacture of synthetic food-grade astaxanthin comprising the following steps:
a) Providing synthetic astaxanthin crystals containing dichloromethane; and
b) Adding methanol to the synthetic astaxanthin in an amount to obtain a suspension of synthetic astaxanthin in methanol, whereby the amount of synthetic astaxanthin in said suspension is in the range of from 5 to 50 weight-%, based on the total weight of the suspension; and
c) bringing the suspension obtained in step b) to a temperature in the range of from 80 to 140°C, whereby the pressure is above atmospheric pressure; and
d) Maintaining the suspension at a temperature in the range of from 80 to 140°C and at a pressure above atmospheric pressure while removing dichloromethane and methanol by distillation until the amount of synthetic astaxanthin in said suspension is in the range of from 25 to 75 weight-%, based on the total weight of the suspension; with the proviso that the amount of synthetic astaxanthin in said suspension is higher than in the starting suspension obtained in step b); and
e) Cooling the synthetic astaxanthin suspension to a temperature in the range of 10 to 35°C; and
f) Optionally filtering off the synthetic astaxanthin crystals; and
g) drying the cooled synthetic astaxanthin suspension obtained in step e) or the filtered synthetic astaxanthin crystals obtained in step f);
whereby during the process the synthetic astaxanthin is not completely solved in the methanol, and whereby a synthetic food-grade astaxanthin with a content of dichloromethane ≤ 250 ppm is obtained.

Step f) is optional, which means that it can be carried out or not.

If step f) is not carried out, the process is as follows: Process 2:
A process for the manufacture of synthetic food-grade astaxanthin comprising the following steps:
i) Providing synthetic astaxanthin crystals containing dichloromethane; and
ii) Adding methanol to the synthetic astaxanthin in an amount to obtain a suspension of synthetic astaxanthin in methanol, whereby the amount of synthetic astaxanthin in said suspension is in the range of from 5 to 50 weight-%, based on the total weight of the suspension; and
iii) bringing the suspension obtained in step b) to a temperature in the range of from 80 to 140°C, whereby the pressure is above atmospheric pressure; and
iv) Maintaining the suspension at a temperature in the range of from 80 to 140°C and at a pressure above atmospheric pressure while removing dichloromethane and methanol by distillation until the amount of synthetic astaxanthin in said suspension is in the range of from 25 to 75 weight-%, based on the total weight of the suspension, with the proviso that the amount of synthetic astaxanthin in said suspension is higher than in the starting suspension obtained in step ii); and
v) Cooling the synthetic astaxanthin suspension to a temperature in the range of 10 to 35°C; and
vi) drying the cooled synthetic astaxanthin suspension obtained in step v) whereby during the process the synthetic astaxanthin is not completely solved in the methanol, and whereby a synthetic food-grade astaxanthin with a content of dichloromethane ≤ 250 ppm is obtained.

If step f) is carried out, the process is as follows: Process 3:
A process for the manufacture of synthetic food-grade astaxanthin comprising the following steps:
A) Providing synthetic astaxanthin crystals containing dichloromethane; and
B) Adding methanol to the synthetic astaxanthin in an amount to obtain a suspension of synthetic astaxanthin in methanol, whereby the amount of synthetic astaxanthin in said suspension is in the range of from 5 to 50 weight-%, based on the total weight of the suspension; and
C) bringing the suspension obtained in step b) to a temperature in the range of from 80 to 140°C, whereby the pressure is above atmospheric pressure; and
D) Maintaining the suspension at a temperature in the range of from 80 to 140°C and at a pressure above atmospheric pressure while removing dichloromethane and methanol by distillation until the amount of synthetic astaxanthin in said suspension is in the range of from 25 to 75 weight-%, based on the total weight of the suspension, with the proviso that the amount of synthetic astaxanthin in said suspension is higher than in the starting suspension obtained in step B); and
E) Cooling the synthetic astaxanthin suspension to a temperature in the range of 10 to 35°C; and
F) filtering off the synthetic astaxanthin crystals; and
G) drying the filtered synthetic astaxanthin crystals obtained in step F); whereby during the process the synthetic astaxanthin is not completely solved in the methanol, and whereby a synthetic food-grade astaxanthin with a content of dichloromethane ≤ 250 ppm is obtained.

Preferably the thus obtained synthetic food-grade astaxanthin has a content of dichloromethane ≤ 100 ppm. Furthermore, such synthetic food-grade astaxanthin has preferably a content of methanol ≤ 250 ppm.

These processes will now be described in more detail below. They are economic and may be performed in an industrial scale. During the process the total amount of astaxanthin is not at any time completely dissolved in the methanol. This is in contrast to a crystallization process where the total amount of astaxanthin is completely dissolved upon heating and re-crystallizes when the temperature is decreased again.

### Step a) / Step i) / Step A)

As starting material synthetic AXN crystals are used. The synthetic AXN crystals are as obtained from any of the chemical syntheses after removing of the solvent dichloromethane, in which the synthesis has been performed. The dichloromethane may have been removed by distillation or by solvent exchange. Preferably the dichloromethane has been removed by a solvent exchange with methanol. In this case the mixture of synthetic astaxanthin and dichloromethane is heated up to a temperature of 38 to 40°C at atmospheric pressure and dichloromethane is distilled off. Simultaneously methanol is added so that the volume of the mixture is kept constant. Then solvent (dichloromethane + methanol) are distilled off until the internal temperature has been raised to 64°C (boiling point of methanol) meaning that only methanol is distilled off, but no dichloromethane any more.

These synthetic AXN crystals still contain dichloromethane which cannot be removed by just prolonging the time for drying these synthetic AXN crystals, even if the temperature and/or the vacuum is/are increased. Typical amounts of remaining dichloromethane in the synthetic AXN crystals are 0.2 to 0.3 weight-%, based on the total weight of the synthetic AXN crystals.

Since the process of the present invention is very effective in removing dichloromethane, it is also possible to use directly **a solution of the synthetic AXN in dichloromethane as starting material** instead of synthetic AXN crystals. Then step step a)/step i)/step A) is as follows:
a)/i)/A) providing a solution of synthetic astaxanthin in dichloromethane.

In this case it is advantageous to first reduce the amount of dichloromethane in the solution by distillation. Preferably this is done whereby simultaneously methanol is added.

Thus, if a solution of synthetic AXN in dichloromethane is used as starting material advantageously steps a2) and a3) are performed after step a)/step i)/step A) and before step b)/step ii)/step B).
a2) optionally adding methanol to the synthetic astaxanthin solution of step a1);
a3) removing dichloromethane by distillation;
   in case step a2) has been performed removing dichloromethane and methanol by distillation.

### Step b) / Step ii) / Step B)

Preferably step b) is carried out in a closed reactor.

Preferably the methanol is added to the synthetic astaxanthin crystals under nitrogen.

### Step c) / Step iii) / Step C)

Preferably the suspension obtained in step b)/step ii)/step B) is brought to a temperature in the range of from 80 to 140°C, more preferably to a temperature in the range of from 100 to 120°C, most preferably to a temperature in the range of from 105 to 115°C. Thereby the pressure increases.

### Step d) / Step iv) / Step D)

When the desired temperature is reached, the temperature is maintained constant and dichloromethane and methanol are removed by distillation until the amount of synthetic AXN in said suspension is in the range of from 25 to 75 weight-% preferably in the range of from 35 to 65 weight-%, even more preferably in the range of 45 to 55 weight-%, based on the total weight of the suspension. Thus, in any case the concentration of the suspension is increased meaning that the amount of the solvent in such suspension is decreased compared to the suspension of step b) / step ii) / step B).

### Step e) / Step v) / Step E)

Preferably the methanolic synthetic astaxanthin suspension, whereby its content of dichloromethane is ≤ 250 ppm, is cooled to a temperature in the range of from 15-30°C, more preferably to a temperature in the range of from 20 to 25°C, leading to synthetic astaxanthin crystals.

### Step f) / Step F)

Optionally the synthetic astaxanthin crystals may be filtered off. This step is preferably carried out. Thus, preferably process 3 is performed.

### Step g) / Step vi) / Step G

The drying of the obtained synthetic AXN crystals is generally carried out at a temperature below 140°C, preferably at a temperature in the range of from 40 to 140°C, and in vacuum. The vacuum is preferably in the range of between 0 to 20 mbara.

In one embodiment of the present invention the drying of the synthetic AXN crystals is carried out at a temperature of 80°C and at a pressure of 20 mbar. In a further embodiment of the present invention the drying of the synthetic AXN crystals is carried out at a temperature in the range of from 55 to 70°C and at a pressure below 20 mbar.

Especially preferred embodiments of the present invention are those processes, whereby one or more of the preferred conditions given above are realized. The most preferred processes of the present invention are those, whereby all preferred conditions given above are realized.

### Oily suspensions

The synthetic food-grade AXN may be admixed with an edible oil to obtain an oily suspension of synthetic food-grade AXN. The edible oil may be selected from the following group consisting of vegetable oils such as corn oil, safflower oil, sunflower oil, middle chain triglycerides (MCT) oil, peanut oil, soybean oil, rapeseed oil, palm oil, palm kernel oil, cotton seed oil, olive oil, coconut oil, and synthetic oils, and any mixture thereof, but is not limited thereto. Especially preferred edible oils are selected from the group consisting of safflower oil, corn oil, sunflower oil, middle chain triglycerides (MCT) oil and any mixture thereof.

Generally, the content of synthetic food-grade AXN in such a suspension is in the range of 0.5 to 20 weight-%, based on the total weight of the suspension. Preferably the content of AXN in such a suspension is in the range of 1.0 to 10 weight-%, based on the total weight of the suspension. More preferably oily suspensions with a content of synthetic food-grade AXN of 5 and 10 weight-%, respectively, are marketed.

The oily suspension may further comprise an antioxidant, especially a fat-soluble antioxidant. An especially preferred fat-soluble antioxidant is dl-alpha-tocopherol. The amount of such anti-oxidant is preferably in the range of 0.1 to 3 weight-%, based on the total weight of the suspension. The amount of the oil is then so much that the amount of all three ingredients (synthetic food-grade AXN; anti-oxidant, oil) sums up to 100 weight-%.

The oily suspension may be directly used as such for the manufacture of dietary supplements in the form capsules, especially in the form of soft-gel capsules.

### Powdery forms

The powdery forms may be used for making tablets.

An especially preferred powdery form is a beadlet comprising the synthetic food-grade astaxanthin as disclosed above encapsulated in a matrix comprising a modified food starch.

The amount of synthetic food-grade astaxanthin in said beadlet is preferably in the range of 0.5 to 20 weight-%, based on the total weight of the beadlet.

The beadlets may further comprise a water-soluble antioxidant (such as sodium ascorbate) and/or a fat-soluble antioxidant (such as DL-alpha-tocopherol), as well as a saccharide as adjuvant. The amount of each of the anti-oxidants is preferably in the range of 0.5 to 3.0 weight-%, based on the total weight of the beadlet.

A particularly preferred modified food starch of this invention has the following formula (I) 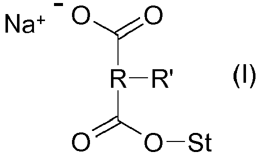 wherein St is a starch, R is an alkylene radical and R' is a hydrophobic group. Preferably R is a lower alkylene radical such as dimethylene or trimethylene. R' may be an alkyl or alkenyl group, preferably having 5 to 18 carbon atoms. A preferred compound of formula (I) is an "OSA-starch" (starch sodium octenyl succinate). The degree of substitution, i.e. the number of esterified hydroxyl groups to the number of free non-esterified hydroxyl groups usually varies in a range of from 0.1% to 10%, preferably in a range of from 0.5% to 4%, more preferably in a range of from 3% to 4%.

The term "OSA-starch" denotes any starch (from any natural source such as corn, waxy maize, waxy corn, wheat, tapioca and potato or synthesized) that was treated with octenyl succinic anhydride (OSA). The degree of substitution, i.e. the number of hydroxyl groups esterified with OSA to the number of free non-esterified hydroxyl groups usually varies in a range of from 0.1% to 10%, preferably in a range of from 0.5% to 4%, more preferably in a range of from 3% to 4%. OSA-starches are also known under the expression "modified food starch".

The term "OSA-starches" encompasses also such starches that are commercially available e.g. from National Starch under the tradenames HiCap 100, Capsul (octenylbutanedioate amylodextrin), Capsul HS, Purity Gum 2000, Clear Gum Co03, UNI-PURE, HYLON VII; from National Starch and Roquette Frères, respectively; from CereStar under the tradename C*EmCap or from Tate & Lyle.

In a preferred embodiment of the present invention a commercially available modified food starch such as e.g. HiCap 100 (from National Starch) and ClearGum Co03 (from Roquette Frères) is used. It is especially advantageous if such a starch or an OSA starch in general is further improved according to a process as disclosed in WO 2007/090614, especially according to a procedure as described in examples 28, 35 and/or 36 of WO 2007/090614.

Thus, in a further improved embodiment of the present invention such a commercially available starch has been centrifuged as an aqueous solution or suspension before use. The centrifugation may be carried out at 1000 to 20000 g depending on the dry mass content of the modified polysaccharide in the aqueous solution or suspension. If the dry mass content of the modified polysaccharide in the aqueous solution or suspension is high, the applied centrifugation force is also high. For example for an aqueous solution or suspension with a dry mass content of the modified polysaccharide of 30 weight-% a centrifugation force of 12000 g may be suitable to achieve the desired separation.

The centrifugation may be carried out at dry matter contents in the range of from 0.1-60 weight %, preferably in the range of from 10-50 weight-%, most preferably in the range of from 15-40 weight-% at temperatures in the range of from 2-99°C, preferably in the range of from 10-75°C, most preferably in the range of from 40-60°C.

The term "saccharide" in the context of the present invention encompasses mono-, di-, oligo- and polysaccharides, as well as any mixtures thereof.

Examples of monosaccharides are fructose, glucose (= dextrose), mannose, galactose, sorbose, as well as any mixtures thereof.

Preferred monosaccharides are glucose and fructose, as well as any mixture thereof.

The term "glucose" in the context of the present invention does not only mean the pure substance, but also a glucose syrup with a DE ≥ 90. This also applies for the other monosaccharides.

The term "dextrose equivalent" (DE) denotes the degree of hydrolysis and is a measure of the amount of reducing sugar calculated as D-glucose based on dry weight; the scale is based on native starch having a DE close to 0 and glucose having a DE of 100.

Examples of disaccharides are saccharose, isomaltose, lactose, maltose and nigerose, as well as any mixture thereof.

An example of an oligosaccharide is maltodextrin.

An example of a polysaccharide is dextrin.

An example of a mixture of mono- and disaccharides is invert sugar (glucose + fructose + saccharose).

Mixtures of mono- and polysaccharides are e.g. commercially available under the tradenames Glucidex IT 47 (from Roquette Frères), Dextrose Monohydrate ST (from Roquette Frères), Sirodex 331 (from Tate & Lyle) and Glucamyl F 452 (from Tate & Lyle).

In a preferred embodiment the amount of the modified food starch is in the range of from 20 to 80 weight-%, more preferably in the range of from 40 to 60 weight-%, and the amount of the saccharide (adjuvant) is in the range of from 5 to 30 weight-%, based on the total amount of the composition.

More preferably the beadlets are manufactured according to the process described below, whereby especially corn starch is used as powder-catch agent. Most preferably the thus resulting beadlets contain from 10 to 25 weight-% of corn starch, based on the total weight of the beadlet.

Preferred are beadlets whose preparation comprises the following steps:
a) providing a modified food starch, a saccharide, a water-/fat-soluble antioxidant and a synthetic food-grade AXN according to the present invention;
b) preparing an aqueous nano-emulsion of said ingredients;
c) converting the nano-emulsion into a beadlet by a powder-catch process.

### Human dietary supplements

For human dietary supplements any synthetic food-grade AXN as described above (with all the preferences given) may be used.

"human dietary supplements" mean dietary supplements to be administered to and to be consumed by humans.

The invention is now further illustrated in the following non-limiting examples.

### Examples

### Examples 1-2: Processes for obtaining synthetic food-grade astaxanthin

### Example 1

120 g of astaxanthin (97%) containing 2000 mg/kg of dichloromethane are introduced in a 1000 ml steel autoclave under inert conditions with 680 ml of methanol. The autoclave is closed, the mixture agitated at 350 rpm (rotations per minute) and heated at 105°C.

Once the wanted internal temperature (105°C) is reached, the solvent is distillated off at a flow rate of 1.35 ml/min during 7 hours (meaning that 567 ml of methanol are distillated off). After that the mixture is cooled to 25°C and diluted with 150 ml methanol. The crystals are filtered off, washed with 150 ml of methanol and dried in a drying oven for 16 hours at 80°C under 20 mbara. 117.4 g of dry astaxanthin are obtained containing 55 mg/kg of dichloromethane and 57 mg/kg of methanol.

### Example 2

120 g of astaxanthin (97%) containing 2000 mg/kg of dichloromethane are introduced in a 1000 ml steel autoclave under inert conditions with 680 ml of methanol. The autoclave is closed, the mixture agitated at 350 rpm and heated at 110°C. Once the wanted internal temperature (110°C) is reached, the solvent is distillated off at a flow rate of 1.4 ml/min during 7 hours (meaning that 588 ml of methanol are distillated off). After that the mixture is cooled to 25°C and diluted with 170 ml of methanol. The crystals are filtered off, washed with 150 ml of methanol and dried in a drying oven for 16 hours at 80°C under 20 mbara. 117.2 g of dry astaxanthin are obtained containing 11 mg/kg of dichloromethane and 140 mg/kg of methanol.

In principle is it possible to perform example 1 or 2 as described above also at any other temperature in the range of from 80 to 140°C instead of 105°C and 110°C, respectively. Such other temperatures are e.g. 80°C, 85°C, 90°C, 95°C, 100°C, 115°C, 120°C, 125°C, 130°C, 135°C, 140°C and any other temperature in between. Example 1 and 2 may especially be repeated at a temperature of 115°C.

### Examples 3-6: powdery forms

### Example 3: Astaxanthin 5% Beadlet Form

10 g of purified crystalline Astaxanthin as obtained in example 1 and 1.7 g of dl-α-tocopherol are dissolved in an appropriate solvent. This solution is added under stirring to a solution of 97.7 g of modified food starch (e.g. Capsul HS), 25.0 g of glucose syrup (e.g. Glucidex IT 47), 3.3 g of sodium ascorbate and 240 g of water at 50 to 60°C.This pre-emulsion is homogenized with a rotor-stator-homogenizer for 20 minutes. Eventually the emulsion is homogenized with a high pressure homogenizer. In the next step the remaining solvent is removed by distillation and the solvent-free emulsion is dried by a standard powder catch process. 140 g of beadlets are obtained with an astaxanthin content of 5.7%.

### Example 4: Astaxanthin 5% Beadlet Form

Example 3 may be repeated by using purified crystalline astaxanthin as obtained in example 2 instead of the purified crystalline astaxanthin as obtained in example 1.

### Example 5: Astaxanthin 10% Beadlet Form

20 g of purified crystalline Astaxanthin as obtained in example 1 and 1.7 g dl-α-tocopherol are dissolved in an appropriate solvent. This solution is added under stirring to a solution of 81.7 g of modified food starch (e.g. Capsul HS), 13.3 g of glucose syrup (e.g. Glucidex IT 47), 8.3 g of sodium ascorbate and 230 g of water at 50 to 60°C.This pre-emulsion is homogenized with a rotor-stator-homogenizer for 20 minutes. Eventually the emulsion is homogenized with a high pressure homogenizer. In the next step the remaining solvent is removed by distillation and the solvent-free emulsion is dried by a standard powder catch process. 140 g of beadlets are obtained with an astaxanthin content of 12.0%.

### Example 6: Astaxanthin 10% Beadlet Form

Example 5 may be repeated by using purified crystalline astaxanthin as obtained in example 2 instead of the purified crystalline astaxanthin as obtained in example 1.

### Examples 7-8: Oily suspensions

### Example 7: Astaxanthin 10% Fluid Suspension

A coarse suspension which contains 60 g of purified crystalline astaxanthin as obtained in example 1, 5.5 g of dl-α-tocopherol and 480 g of safflower oil is prepared under mixing at 45°C. For particle size reduction, this coarse suspension is pumped continuously through an agitated ball mill (diameter of the zirconiumdioxide pearls: 0.4 mm, Dispermate SL C12 ball mill; VMA-Getzmann GmbH, Germany) until the desired average particle size (< 2 microns) is achieved. After 140 minutes of milling time, 450 g of an oily suspension results with an astaxanthin content of 10.7% and an average particle size of 1.8 microns (measured with Malvern Mastersizer 3000 equipment, Fraunhofer presentation).

### Example 8: Astaxanthin 10% Fluid Suspension

Example 7 may be repeated by using purified crystalline astaxanthin as obtained in example 2 instead of the purified crystalline astaxanthin as obtained in example 1.

## Claims

1. A process for the manufacture of synthetic food-grade astaxanthin with a content of dichloromethane ≤ 250 ppm comprising the following steps:
a) Providing synthetic astaxanthin crystals containing dichloromethane; and
b) Adding methanol to the synthetic astaxanthin in an amount to obtain a suspension of synthetic astaxanthin in methanol, whereby the amount of synthetic astaxanthin in said suspension is in the range of from 5 to 50 weight-%, based on the total weight of the suspension; and
c) bringing the suspension obtained in step b) to a temperature in the range of from 80 to 140°C, whereby the pressure is above atmospheric pressure; and
d) Maintaining the suspension at a temperature in the range of from 80 to 140°C and at a pressure above atmospheric pressure while removing dichloromethane and methanol by distillation until the amount of synthetic astaxanthin in said suspension is in the range of from 25 to 75 weight-%, based on the total weight of the suspension, with the proviso that the amount of synthetic astaxanthin in said suspension is higher than in the starting suspension obtained in step b); and
e) Cooling the synthetic astaxanthin suspension to a temperature in the range of 10 to 35°C; and
f) Optionally filtering off the synthetic astaxanthin crystals; and
g) drying the cooled synthetic astaxanthin suspension obtained in step e) or the filtered synthetic astaxanthin crystals obtained in step f); whereby during the process the synthetic astaxanthin is not completely solved in the methanol, and whereby a synthetic food-grade astaxanthin with a content of dichloromethane ≤ 250 ppm is obtained.

2. The process according to claim 1, whereby in step c) the suspension is brought to a temperature in the range of from 100 to 120°C, preferably to a temperature in the range of from 105 to 115°C.

3. The process according to claim 1 or 2, whereby in step d) dichloromethane and methanol are removed by distillation until the amount of synthetic AXN in said suspension is in the range of from 35 to 65 weight-%, preferably until the amount of synthetic AXN in said suspension is in the range of from 45 to 55 weight-%, based on the total weight of the suspension.

4. A process for the manufacture of synthetic food-grade astaxanthin with a content of dichloromethane ≤ 250 ppm comprising the following steps:
a) Providing a solution of synthetic astaxanthin in dichloromethane; and
b) Adding methanol to the synthetic astaxanthin solution in an amount to obtain a suspension of synthetic astaxanthin in methanol, whereby the amount of synthetic astaxanthin in said suspension is in the range of from 5 to 50 weight-%, based on the total weight of the suspension; and
c) bringing the suspension obtained in step b) to a temperature in the range of from 80 to 140°C, whereby the pressure is above atmospheric pressure; and
d) Maintaining the suspension at a temperature in the range of from 80 to 140°C and at a pressure above atmospheric pressure while removing dichloromethane and methanol by distillation until the amount of synthetic astaxanthin in said suspension is in the range of from 25 to 75 weight-%, based on the total weight of the suspension, with the proviso that the amount of synthetic astaxanthin in said suspension is higher than in the starting suspension obtained in step b); and
e) Cooling the synthetic astaxanthin suspension to a temperature in the range of 10 to 35°C; and
f) Optionally filtering off the synthetic astaxanthin crystals; and
g) drying the cooled synthetic astaxanthin suspension obtained in step e) or the filtered synthetic astaxanthin crystals obtained in step f);
whereby during the process the synthetic astaxanthin is not completely solved in the methanol, and whereby a synthetic food-grade astaxanthin with a content of dichloromethane ≤ 250 ppm is obtained.

5. The process according to claim 4, whereby in step c) the suspension is brought to a temperature in the range of from 100 to 120°C, preferably to a temperature in the range of from 105 to 115°C.

6. The process according to claim 4 or 5, whereby in step d) dichloromethane and methanol are removed by distillation until the amount of synthetic AXN in said suspension is in the range of from 35 to 65 weight-%, preferably until the amount of synthetic AXN in said suspension is in the range of from 45 to 55 weight-%, based on the total weight of the suspension.

7. The process according to claim 4, whereby after step a) and before step b) the following steps are performed:
a2) optionally adding methanol to the synthetic astaxanthin solution of step a); and
a3) removing dichloromethane by distillation;
in case step a2) has been performed by removing dichloromethane and methanol by distillation.

## Patentansprüche

1. Verfahren zur Herstellung von synthetischem Astaxanthin in Lebensmittelqualität mit einem Gehalt an Dichlormethan ≤ 250 ppm, welches die folgenden Schritte umfasst:
a) Bereitstellen von synthetischen Astaxanthinkristallen, die Dichlormethan enthalten; und
b) Zufügen von Methanol zu dem synthetischen Astaxanthin in einer Menge, um eine Suspension von synthetischem Astaxanthin in Methanol zu erhalten, wobei die Mange an synthetischem Astaxanthin in der Suspension im Bereich von 5 bis 50 Gew.% liegt, bezogen auf das Gesamtgewicht der Suspension; und
c) Bringen der in Schritt b) erhaltenen Suspension auf eine Temperatur im Bereich von 80 bis 140°C, wobei der Druck oberhalb von atmosphärischem Druck liegt; und
d) Halten der Suspension bei einer Temperatur im Bereich von 80 bis 140°C und einem Druck oberhalb von atmosphärischem Druck, während Dichlormethan und Methanol mittels Destillation entfernt werden, bis die Menge an synthetischem Astaxanthin in der Suspension im Bereich von 25 bis 75 Gew.% liegt, bezogen auf das Gesamtgewicht der Suspension, mit der Maßgabe, dass die Menge an synthetischem Astaxanthin in der Suspension höher als in der in Schritt b) erhaltenen Ausgangssuspension ist; und
e) Kühlen der synthetischen Astaxanthinsuspension auf eine Temperatur im Bereich von 10 bis 35°C; und
f) gegebenenfalls Abfiltrieren der synthetischen Astaxanthinkristalle; und
g) Trocknen der gekühlten synthetischen Astaxanthinsuspension, die in Schritt e) erhalten wurde, oder der filtrierten synthetischen Astaxanthinkristalle, die in Schritt f) erhalten wurden;
wobei das synthetische Astaxanthin während des Verfahrens nicht vollständig in dem Methanol gelöst wird, und wobei ein synthetisches Astaxanthin in Lebensmittelqualität mit einem Gehalt an Dichlormethan ≤ 250 ppm erhalten wird.

2. Verfahren nach Anspruch 1, wobei die Suspension in Schritt c) auf eine Temperatur im Bereich von 100 bis 120°C gebracht wird, vorzugsweise auf eine Temperatur im Bereich von 105 bis 115°C.

3. Verfahren nach Anspruch 1 oder 2, wobei in Schritt d) Dichlormethan und Methanol mittels Destillation entfernt werden, bis die Menge an synthetischem AXN in der Suspension im Bereich von 35 bis 65 Gew.% liegt, vorzugsweise bis die Menge an synthetischem AXN in der Suspension im Bereich von 45 bis 55 Gew.% liegt, bezogen auf das Gesamtgewicht der Suspension.

4. Verfahren zur Herstellung von synthetischem Astaxanthin in Lebensmittelqualität mit einem Gehalt an Dichlormethan ≤ 250 ppm, welches die folgenden Schritte umfasst:
a) Bereitstellen einer Lösung von synthetischem Astaxanthin in Dichlormethan; und
b) Zufügen von Methanol zu der Lösung des synthetischen Astaxanthins in einer Menge, um eine Suspension von synthetischem Astaxanthin in Methanol zu erhalten, wobei die Menge an synthetischem Astaxanthin in der Suspension im Bereich von 5 bis 50 Gew.% liegt, bezogen auf das Gesamtgewicht der Suspension; und
c) Bringen der in Schritt b) erhaltenen Suspension auf eine Temperatur im Bereich von 80 bis 140°C, wobei der Druck oberhalb von atmosphärischem Druck liegt; und
d) Halten der Suspension bei einer Temperatur im Bereich von 80 bis 140°C und einem Druck oberhalb von atmosphärischem Druck, während Dichlormethan und Methanol mittels Destillation entfernt werden, bis die Menge an synthetischem Astaxanthin in der Suspension im Bereich von 25 bis 75 Gew.% liegt, bezogen auf das Gesamtgewicht der Suspension, mit der Maßgabe, dass die Menge an synthetischem Astaxanthin in der Suspension höher als in der in Schritt b) erhaltenen Ausgangssuspension ist; und
e) Kühlen der synthetischen Astaxanthinsuspension auf eine Temperatur im Bereich von 10 bis 35°C; und
f) gegebenenfalls Abfiltrieren der synthetischen Astaxanthinkristalle; und
g) Trocknen der gekühlten synthetischen Astaxanthinsuspension, die in Schritt e) erhalten wurde, oder der filtrierten synthetischen Astaxanthinkristalle, die in Schritt f) erhalten wurden;
wobei während des Verfahrens das synthetische Astaxanthin nicht vollständig in dem Methanol gelöst wird, und wobei ein synthetisches Astaxanthin in Lebensmittelqualität mit einem Gehalt an Dichlormethan ≤ 250 ppm erhalten wird.

5. Verfahren nach Anspruch 4, wobei die Suspension in Schritt c) auf eine Temperatur im Bereich von 100 bis 120°C gebracht wird, vorzugsweise auf eine Temperatur im Bereich von 105 bis 115°C.

6. Verfahren nach Anspruch 4 oder 5, wobei in Schritt d) Dichlormethan und Methanol durch Destillation entfernt werden, bis die Menge an synthetischem AXN in der Suspension im Bereich von 35 bis 65 Gew.% liegt, vorzugsweise bis die Menge an synthetischem AXN in der Suspension im Bereich von 45 bis 55 Gew.% liegt, bezogen auf das Gesamtgewicht der Suspension.

7. Verfahren nach Anspruch 4, wobei nach Schritt a) und vor Schritt b) die folgenden Schritte durchgeführt werden:
a2) gegebenenfalls Hinzufügen von Methanol zu der synthetischen Astaxanthinlösung von Schritt a); und
a3) Entfernen von Dichlormethan durch Destillation; falls Schritt a2) durchgeführt worden ist durch Entfernen von Dichlormethan und Methanol mittels Destillation.

## Revendications

1. Procédé de fabrication d'astaxanthine synthétique de qualité alimentaire ayant une teneur en dichlorométhane ≤ 250 ppm comprenant les étapes suivantes :
a) fourniture de cristaux d'astaxanthine synthétique contenant du dichlorométhane ; et
b) ajout de méthanol à l'astaxanthine synthétique en une quantité pour obtenir une suspension d'astaxanthine synthétique dans du méthanol, dans lequel la quantité d'astaxanthine synthétique dans ladite suspension est dans la plage de 5 à 50 % en poids, sur la base du poids total de la suspension ; et
c) chauffage de la suspension obtenue dans l'étape b) à une température dans la plage de 80 à 140°C, dans lequel la pression est supérieure à la pression atmosphérique ; et
d) maintien de la suspension à une température dans la plage de 80 à 140°C et à une pression supérieure à la pression atmosphérique tout en éliminant le dichlorométhane et le méthanol par distillation jusqu'à ce que la quantité d'astaxanthine synthétique dans ladite suspension soit dans la plage de 25 à 75% en poids, sur la base du poids total de la suspension, à condition que la quantité d'astaxanthine synthétique dans ladite suspension soit supérieure à celle dans la suspension de départ obtenue dans l'étape b) ; et
e) refroidissement de la suspension d'astaxanthine synthétique à une température dans la plage de 10 à 35°C; et
f) facultativement séparation par filtration des cristaux d'astaxanthine synthétique ; et
g) séchage de la suspension d'astaxanthine synthétique refroidie obtenue dans l'étape e) ou des cristaux filtrés d'astaxanthine synthétique obtenus dans l'étape f) ; dans lequel, pendant le procédé, l'astaxanthine synthétique n'est pas complètement dissoute dans le méthanol, et dans lequel une astaxanthine synthétique de qualité alimentaire ayant une teneur en dichlorométhane ≤ 250 ppm est obtenue.

2. Procédé selon la revendication 1, dans lequel, dans l'étape c) la suspension est amenée à une température dans la plage de 100 à 120°C, de préférence à une température dans la plage de 105 à 115°C.

3. Procédé selon la revendication 1 ou 2, dans lequel, dans l'étape d), le dichlorométhane et le méthanol sont éliminés par distillation jusqu'à ce que la quantité d'AXN synthétique dans ladite suspension soit dans la plage de 35 à 65% en poids, de préférence jusqu'à ce que la quantité d'AXN synthétique dans ladite suspension soit dans la plage de 45 à 55% en poids, sur la base du poids total de la suspension.

4. Procédé de fabrication d'astaxanthine synthétique de qualité alimentaire ayant une teneur en dichlorométhane ≤ 250 ppm comprenant les étapes suivantes :
a) fourniture d'une solution d'astaxanthine synthétique dans le dichlorométhane ; et
b) ajout de méthanol à la solution d'astaxanthine synthétique en une quantité adaptée pour obtenir une suspension d'astaxanthine synthétique dans du méthanol, dans lequel la quantité d'astaxanthine synthétique dans ladite suspension est dans la plage de 5 à 50 % en poids, sur la base du poids total de la suspension ; et
c) chauffage de la suspension obtenue dans l'étape b) à une température dans la plage de 80 à 140°C, dans lequel la pression est supérieure à la pression atmosphérique ; et
d) maintien de la suspension à une température dans la plage de 80 à 140°C et à une pression supérieure à la pression atmosphérique tout en éliminant le dichlorométhane et le méthanol par distillation jusqu'à ce que la quantité d'astaxanthine synthétique dans ladite suspension soit dans la plage de 25 à 75% en poids, sur la base du poids total de la suspension, à condition que la quantité d'astaxanthine synthétique dans ladite suspension soit supérieure à celle dans la suspension de départ obtenue dans l'étape b) ; et
e) refroidissement de la suspension d'astaxanthine synthétique à une température dans la plage de 10 à 35°C ; et
f) facultativement, séparation par filtration des cristaux d'astaxanthine synthétique ; et
g) séchage de la suspension d'astaxanthine synthétique refroidie obtenue dans l'étape e) ou des cristaux d'astaxanthine synthétique filtrés obtenus dans l'étape f) ;
dans lequel, pendant le procédé, l'astaxanthine synthétique n'est pas complètement dissoute dans le méthanol, et dans lequel une astaxanthine synthétique de qualité alimentaire ayant une teneur en dichlorométhane ≤ 250 ppm est obtenue.

5. Procédé selon la revendication 4, dans lequel, dans l'étape c), la suspension est amenée à une température dans la plage de 100 à 120°C, de préférence à une température dans la plage de 105 à 115°C.

6. Procédé selon la revendication 4 ou 5, dans lequel, dans l'étape d), le dichlorométhane et le méthanol sont éliminés par distillation jusqu'à ce que la quantité d'AXN synthétique dans ladite suspension soit dans la plage de 35 à 65% en poids, de préférence jusqu'à ce que la quantité d'AXN synthétique dans ladite suspension soit dans la plage de 45 à 55% en poids, sur la base du poids total de la suspension.

7. Procédé selon la revendication 4, dans lequel, après l'étape a) et avant l'étape b) les étapes suivantes sont conduites :
a2) facultativement, ajout de méthanol à la solution d'astaxanthine synthétique de l'étape a); et
a3) élimination du dichlorométhane par distillation ;
dans le cas où l'étape a2) a été conduite en éliminant le dichlorométhane et le méthanol par distillation.
